# EUROPEAN PATENT APPLICATION

(11) **EP 1 356 810 A1**
(43) Date of publication of application: **29.10.2003**
(21) Application number: 01273986.8
(22) Date of filing: 18.12.2001
(51) Int. Cl.: A61K 31/185, A61K 31/255, A61P 43/00, A61P 1/16

(54) **TNFa PRODUCTION INHIBITORS**

(30) Priority: 27.12.2000 JP 2000397522
(71) Applicant: Ajinomoto Co., Inc., Tokyo 104-8315 (JP)
(72) Inventor: ISHIZAKI, S., c/o Ajinomoto Co., Inc., Kawasaki-shi, Kanagawa 210-0801 (JP); SONAKA, Ichiro, c/o Ajinomoto Co., Inc., Kawasaki-shi, Kanagawa 210-0801 (JP); IINO, Yukio, c/o Ajinomoto Co., Inc., Kawasaki-shi, Kanagawa 210-0801 (JP)
(74) Representative: Nash, David Allan
(86) International application number: JP0111112
(87) International publication number: WO02074299

(57) **Abstract**

There is provided a medicinal composition containing aminomethanesulfonic acid (including that in a form of salt or ester) as an active ingredient. Since it has an excellent effect of inhibiting the production of TNFα as compared with glycine which has been proposed already, the composition is expected as a drug for liver diseases. There are also provided a use of the above-mentioned active ingredient for drugs, a method for inhibiting the production of TNFα and, particularly, a method of treatment, amelioration and/or prevention of liver diseases utilizing the above method.

## Description

### Technical Field

This invention relates to a novel medicinal composition. To be more specific, it relates to a medicinal composition containing aminomethanesulfonic acid (including that in a form of a derivative which is able to be converted to aminomethanesulfonic acid in a living body such as salt, ester, etc.) as an active ingredient and, more particularly, it relates to an inhibitor for the production of TNFα. It can be used as a drug such as a drug for liver diseases. This invention further relates to a method for inhibiting the production of TNFα, particularly a method for treatment, amelioration and/or prevention of liver diseases and to a use of the above-mentioned active ingredient for a medicinal composition, preferably an inhibitor for the production of TNFα or a drug for liver diseases, etc. as well.

Up to now, glycine has been proposed as an ingredient for drugs where an inhibiting action for the production of TNFα is utilized, and the effect therefor can now be significantly improved in this invention as compared with the case of glycine.

### Background Art

Glycine, alanine and serine or, particularly, glycine have/has been proposed as ingredient(s) for drugs or nutritive agents (nutritional preparation) for decreasing the tumor necrosis factor (TNF) level in patients where the level increases beyond the range of mediation of physiological homeostasis and local inflammation (refer to WO 96/25861 and JP-A-11-501301). However, the effect is not sufficient and there is a demand for development of drugs where the effect is improved.

### Object of Invention, and Problem before Invention

An object of this invention is to develop a better inhibitor for the production of TNFα and to use it as drug(s) (drug for liver diseases, etc.) utilizing the action thereof.

### Disclosure of Invention

In order to resolve the above-mentioned problem, the present inventors have carried out intensive investigations and, as a result, they have found that aminomethanesulfonic acid is quite excellent in an effect of inhibiting the production of TNFα. On the basis of such a finding, the present invention has been achieved.

Thus, this invention relates to a medicinal composition which is characterized in containing aminomethanesulfonic acid as an active ingredient. As will be mentioned later, the aminomethanesulfonic acid may be in a form of various derivatives such as salt and ester.

When it is used as a drug (medicament), its excellent inhibiting action for the production of TNFα is utilized and, therefore, it can be used as an inhibitor for the production of TNFα. It is particularly appropriate as a drug for liver diseases.

The above active ingredient used for this invention is aminomethanesulfonic acid and it includes a form of derivative(s) which are able to be converted to aminomethanesulfonic acid in a living body, particularly in a living body of human being, such as a salt thereof, an ester thereof, and the like. The ester may be represented by the following general formula (1).

H₂N - CH₂ - SO₃R (1)

In the above formula, R may represent a substituent which constitutes the ester, the R being able to be converted to a hydrogen atom in a living body, particularly in a human body. With regard to such a substituent, an alkyl group having 1 to 6 carbons may be preferably exemplified.

On the other hand, the salt (a sulfonate) may be a salt which is able to be converted to a free substance in a living body, particularly in a human body, and sodium salt, potassium salt, calcium salt, magnesium salt, ammonium salt (including a salt with an amine such as methylamine, dimethylamine, methylethylamine, triethylamine, etc.) and the like may be preferably exemplified.

It is an essential condition for the medicinal composition of this invention to contain the above-mentioned active ingredient and, so far as the advantage of this invention that an inhibiting action for the production of TNFα is aimed is not disturbed, it is also possible to use other active ingredient(s) as the ingredient of the drug in either a mixed form or a combined form. In the manufacture of a medicinal preparation for the medicinal composition in the present invention, it is further possible to appropriately select and use additional ingredients which are necessary for the manufacture of medicinal (pharmaceutical) preparations.

As another embodiments, this invention also relates to a method for inhibiting the production of TNFα which is characterized in administering the aminomethanesulfonic acid to a living subject (body), and to a method for treatment, amelioration and/or prevention of liver diseases which is characterized in administering the aminomethanesulfonic acid to a living body (the aminomethanesulfonic acid may be in a form of salt or ester in both of the above-mentioned embodiments).

With regard to a mode for the administration as such, it is possible to adopt a thing which is in a form of the above-mentioned medicinal composition of the present invention.

As a still another embodiment, this invention further relates to a use of aminomethanesulfonic acid for a medicinal composition, particularly an inhibitor for the production of TNFα, a drug for liver diseases, etc (where the aminomethanesulfonic acid may be in a form of salt or ester).

Such a medicinal composition is as same as that illustrated hereinabove already for the present invention.

### Brief Description of Drawings

### [Fig. 1]

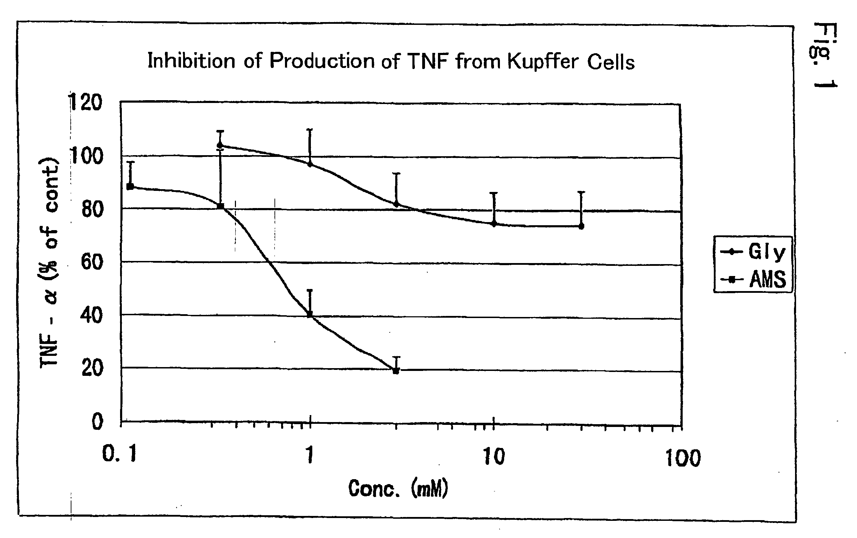

Fig. 1 diagrams the result where the amount of production of TNFα is measured in Example 1.

AMS: aminomethanesulfonic acid; Gly: glycine.

### [Fig. 2]

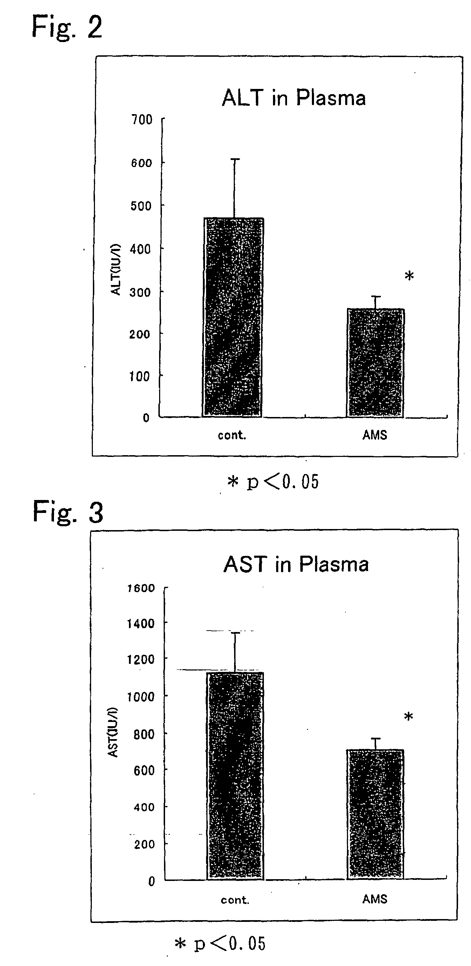

Fig. 2 shows the result of measurement of ALT in plasma obtained in Example 2.

AMS: aminomethanesulfonic acid; * p < 0.05.

### [Fig. 3]

Fig. 3 shows the result of measurement of AST in plasma obtained in Example 2.

AMS: aminomethanesulfonic acid; * p < 0.05.

### Mode for Carrying Out Invention

As hereunder, a mode for carrying out the present invention will be illustrated.

The medicinal composition (medicament composition) of this invention is preferably used as a drug using an action of inhibiting the production of TNFα (an inhibitor for the production of TNFα). It is suitable for prevention, amelioration and/or treatment of liver diseases such as alcoholic, viral or drug-induced hepatitis, hepatic fibrosis, cirrhosis and fulminant hepatitis, inflammatory intestinal diseases, pancreatitis, arthritis, arteriosclerosis, sepsis, ischemic reperfusion injury, etc. It is particularly useful for prevention, amelioration and/or treatment of liver diseases.

The aminomethanesulfonic acid which is an active ingredient used in this invention is a known compound and can be easily prepared although it is convenient to use by purchasing it from a market (that manufactured by Tokyo Kasei Kogyo K. K., etc.).

In the formation of ester or salt when the aminomethanesulfonic acid is used in a form of ester or salt, aimed ester or salt can be easily prepared by utilizing a known art where a sulfonate (sulfonic acid ester) is prepared from a sulfonic acid or by utilizing a salt formation step by addition of alkali.

When the active ingredient is used, for example, as a drug for liver diseases, the form of the medicinal preparation in the medicinal composition is not particularly limited but may be either an oral preparation or a parenteral preparation (such as an injection preparation).

With regard to the dose of the active ingredient, although it depends upon symptom of the patient, dosage form, etc., there may be used preferably about (approximately) 1 mg to 10 g, more preferably about (approximately) 10 mg to 5 g and, and further more preferably, about (approximately) 100 mg to 1 g per day in the case of aminomethanesulfonic acid (granular preparation/oral preparation) for a patient suffering, for example, from liver diseases. When it is used as an injection preparation for intravenous administration, the dose of about (approximately) from one-twentieth to one-half of the above active ingredient used for the above oral preparation is sufficient.

Although a dosage form containing at least the essential active ingredient (the above-mentioned aminomethanesulfonic acid) used for this invention can be administered to patients, it is also possible to use a medicinal composition achieving an inhibitory effect for the production of TNFα where a medicinal ingredient which is other than the active ingredient is further contained therein or combined therewith and, needless to say, such a case is also covered by this invention.

In the manufacture of the medicinal preparation in the medicinal composition, various pharmacologically-acceptable substances for medicinal preparations may be contained (as auxiliary agents, etc.). Such substances for medicinal preparations may be appropriately selected depending upon the dosage form of the preparation. For example, they are filler, diluent, additive, disintegrating agent, binder, coating agent, lubricant, gliding agent, lubricating agent, flavoring agent, sweetener, solubilizing agent, spice, dye, nutrient such as vitamin and other additives which are suitable for being contained in the preparation. Further specific examples of the substances for medicinal preparations are magnesium carbonate, titanium dioxide, saccharide(s) such as lactose, mannitol, etc., talc, milk protein, gelatin, starch, cellulose and derivatives thereof, animal and plant oil(s), polyethylene glycol and solvent(s) such as aseptic water (sterilized water) and mono- and polyhydric alcohol (e.g., glycerol).

The drug (medicinal composition) of this invention can be prepared in various medicinal preparation forms or various dosage forms for oral administration, intraperitoneal administration, percutaneous administration, inhalation administration, etc. In order to make the medicinal ingredient used in this invention into such various medicinal preparation forms, methods which have been known or which will be developed in future may be appropriately adopted.

With regard to various forms of medicinal preparations as such, there may be exemplified appropriate preparation forms in solid or in solution such as granule, powder, coated tablet, tablet, diluted powder (powder medicine), (micro)capsule, suppository, syrup, juice, suspension, emulsion, dropping agent, solution for injection, preparation for extending the release of the active ingredient, and the like.

It goes without saying that the drug of this invention in the above-exemplified dosage forms contains at least the above-mentioned ingredient (aminomethanesulfonic acid) in an amount which is effective for achieving the aimed medicinal effect.

As mentioned above, as another embodiment(s), this invention also relates to a method for inhibiting the production of TNFα which is characterized in administering aminomethanesulfonic acid to a living subject (body), and relates to a method for treatment, amelioration and/or prevention of liver diseases which is characterized in administering aminomethanesulfonic acid to a living body (in both of the above cases, the aminomethanesulfonic acid may be in a form of salt, ester or the like). As a still another embodiment, this invention further relates to a use of aminomethanesulfonic acid for a medicinal composition, particularly an inhibitor for the production of TNFα, a drug for liver diseases, etc. (the aminomethanesulfonic acid may be in a form of salt, ester or the like).

With regard to the invention(s) for the above embodiments as such, they may be easily carried out on the basis of the above-mentioned illustration for the medicinal composition of this invention or of the Examples which will be mentioned later or may be carried out by referring to the already known art if necessary.

### Examples

As hereunder, this invention will be illustrated in detail by way of Examples and Comparative Examples although this invention is not limited to those examples.

### (Example 1)

Male rat of SD strain (body weight: 200∼300 g) was subjected to laparotomy under anesthetization with Nembutal, a collagenase solution was perfused from portal vein, then liver was excised and Kupffer cells were separated by an elutriator method. They were adjusted to an extent of 5 × 10⁵ cells/ml, each 100 µ/well thereof were spread on a 96-well microplate and incubation was carried out for 48 hours using E-MEM (Eagle-MEM) and 10% FCS (fetal calf serum) to conduct an experiment. LPS (lipopoly saccharide) (10 µg/ml) and aminomethanesulofonic acid (AMS) or glycine were added at the same time thereto so as to make their concentrations 0.1 to 3 mM or 0.3 to 30 mM, respectively and the amount of TNFα produced in a supernatant liquid after incubation for 4 hours was measured by an ELISA (enzyme-linked immunosorbent assay). The result is shown in Fig. 1.

It is apparent from the result of Fig. 1 that aminomethanesulfonic acid inhibited the production of TNFα in a considerably strong manner as compared with glycine and its use in various kinds of drugs as an inhibitor for the production of TNFα can be expected.

### (Example 2)

### Action of aminomethanesulfonic acid (AMS) to necrosis of hepatocytes

### (Method of Experiment)

Male rat of SD strain of 6 weeks age (150 g) was introduced, subjected to a preliminary breeding by giving CRF-1 (a feed manufactured by Oriental Yeast) and water for six days, fasted for one night and used for the experiment. During the fasting, a 10% aqueous solution of glucose was freely taken by the rat for preventing the lowering of blood-sugar level. D-galactosamine (600 mg/kg) adjusted to pH 6.8 was intraperitoneally administered using a 30% physiological saline solution. After 24 hours from administration of D-galactosamine, 700 µl of blood were collected from subclavian vein under anesthetization with ether and AST (aspartate aminotransferase) and ALT (alanine aminotransferase) in plasma were measured by a Fuji Dry Chem (an automated biochemical measuring apparatus for blood manufactured by Fuji Photo-Film). One hour before the administration of D-galactosamine, AMS was orally administered as a drug to be tested using a 0.3% aqueous solution of carboxymethyl cellulose (CMC).

| (Administered Groups) | |
|---|---|
| Group 1: 0.3% CMC | n = 8; |
| Group 2: AMS (1.0 g/kg)/0.3% CMC | n = 8. |

| (Result of Evaluation) | |
|---|---|
| | |

Results of the above measurement are shown in Fig. 2 and Fig. 3.

From those results, it was noted that, in the group administered with aminomethanesulfonic acid, a rise in AST and that in ALT in the plasma after 24 hours from administration of D-galactosamine were significantly inhibited.

### Effect of Invention

In accordance with the present invention, an inhibitor for the production of TNFα containing aminomethanesulfonic acid (which may be in a form of salt, ester, etc.) as an active ingredient is provided and is able to be used for drugs (a drug for liver diseases, etc.) utilizing its action of inhibiting the production of TNFα.

There are also provided a use of the above-mentioned active ingredient for drugs, a method for inhibiting the production of TNFα and, particularly, a method for treatment, amelioration and/or prevention of liver diseases utilizing such a method.

Accordingly, this invention is quite useful in industry, particularly in the fields of medical treatment, drugs, etc.

## Claims

1. A medicinal composition which is **characterized in** containing aminomethanesulfonic acid as an active ingredient where the aminomethanesulfonic acid may be in a form of salt or ester.

2. The medicinal composition according to claim 1, wherein the ester is represented by the following formula (1)
H₂N - CH₂ - SO₃R (1)
in which R is an alkyl group having 1 to 6 carbon(s).

3. The medicinal composition according to claim 1 or 2, which is an inhibitor for the production of TNFα.

4. The medicinal composition according to any of claims 1 to 3, which is a drug for liver diseases.

5. The medicinal composition according to claim 1, wherein the salt is selected from sodium salt, potassium salt, calcium salt, magnesium salt and ammonium salt.

6. The medicinal composition according to any of claims 1 to 5, which is in a form of solution or in a form of any of granules, tablets and powders.

7. A method for inhibiting the production of TNFα, **characterized in that**, aminomethanesulfonic acid is administered to a living body where the aminomethanesulfonic acid may be in a form of salt or ester.

8. A method for treatment, amelioration and/or prevention of liver diseases, **characterized in that**, aminomethanesulfonic acid is administered to a living body where the aminomethanesulfonic acid may be in a form of salt or ester.

9. The method according to claim 7 or 8, wherein the form for such an administration is in a form of the medicinal composition mentioned in any of claims 1 to 6.

10. A use of aminomethanesulfonic acid for a medicinal composition where the aminomethanesulfonic acid may be in a form of salt or ester.

11. The use according to claim 10, wherein the medicinal composition is an inhibitor for the production of TNFα or a drug for liver diseases.

12. The use according to claim 10, wherein the medicinal composition is in a form of the medicinal composition mentioned in any of claims 1 to 6.
